# EUROPEAN PATENT APPLICATION

(11) **EP 4 194 523 A1**
(43) Date of publication of application: **14.06.2023**
(21) Application number: 21383116.7
(22) Date of filing: 07.12.2021
(51) Int. Cl.: C09J 4/00, A61L 24/00, C08F 222/32

(54) **CYANOACRYLATE ADHESIVE COMPOSITION**

(71) Applicant: Cuantum Medical Cosmetics, S.L., 08193 Cerdanyola del Vallès (Barcelona) (ES)
(72) Inventor: AGUILERA CORROCHANO, Jordi, 08208 Sabadell (ES); ORTEGO HUGUET, Sara, 07712 Maó (ES); SÁEZ FRAILE, Rubén, 08021 Barcelona (ES); CLOSA FERRER, Guillem, 08172 Sant Cugat del Vallès (ES); SANTIAGO RANDO, Irene, 19005 Guadalajara (ES); CORREA ÁLVAREZ, Adán, 18610 Lobres (ES); DÍEZ GONZÁLEZ, Irene, 53007 Tarragona (ES); PUJOL NOGUERA, Ferran, 08635 Sant Esteve Sesrovires (ES)
(74) Representative: Araujo, Daniel

(57) **Abstract**

The present invention refers to a cyanoacrylate adhesive composition comprising a monomer composition comprising cyanoacrylate monomer, multicyanoacrylate monomer, preferably bis-cyanoacrylate monomer, and unsaturated carboxylic acid anhydride. It also relates to a syringe or a cartridge comprising said composition, to the use of said composition for bonding substrates to a method for bonding substrates using said composition, and said composition for use as tissue adhesive (bioadhesive) and biosealant.

## Description

### Field of the invention

The present invention relates to the field of cyanoacrylate adhesive composition, in particular cyanoacrylate compositions with improved thermal bonding performance and chemical/hydrolysis resistance.

### Background art

Cyanoacrylate (CA) is the generic name for a family of resistant fast acting adhesives based on esters of 2-cyanoacrylic acid. The structure of the monomer is as follows: 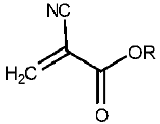 wherein R is generally an alkyl group such as, for example, methyl, ethyl, butyl or 2-octyl.

Such compounds are well known for some time, as disclosed, for example, in S. Ebnesajjad Ed., Adhesives Technology Handbook, William Andrew, Norwich, 2008.

These CA compounds are ethylene monomers 1,1-disubstituted with electron withdrawing groups, so that they are highly reactive towards nucleophiles for its electron deficiency. Their structure allows an extremely easy polymerisation thereof initiated by any nucleophilic or basic species which is inherently present in the medium or substrate to be bonded.

It is known that "instant adhesives" based on alkyl CA esters, while they are effective bonding agents for a wide variety of materials, once cured, exhibit decreased performance at higher temperatures (above 80 °C). This fact could easily be explained in terms of the structure of the adhesive layer formed, containing quaternary carbon atoms in the main chain.

CA polymers are thermoplastic and therefore they soften themselves as the temperature reaches their Tg; beyond this Tg, they begin to retro-polymerize. It is of practical interest to overcome, at least to some degree, this disadvantage to increase the versatility of the CA adhesives.

As regards CA adhesives, the only possible way toward increased heat resistance is the formation of crosslinks which modifies the polymer Tg, thereby reducing its ability to depolymerize.

The operating temperature restrictions have prompted several attempts to improve these limitations, so far, two main approaches have been described to the purpose by adding either crosslinking agents or heat-resistant adhesion promoters to the CA monomers.

In prior art, different approaches have been disclosed to improve the thermal bonding performance and/or the hydrolysis resistance of CA compositions.

The use of different anhydrides or derivatives has been proposed in the art to solve said problems: maleic anhydride in British patent application GB-A-1365195, phthalic anhydride in European patent application EP-A-0058187, benzophenone tetracarboxylic acid or anhydride in European patent application EP-A-0137849, substituted or unsubstituted aliphatic carboxylic acids having three or more carboxyl groups, anhydrides thereof, partial esters thereof, aromatic polycarboxylic acids having three or more carboxyl groups and anhydrides thereof in US4196271, and maleimides in US4490515.

In European patent application EP-A-0144178 it is disclosed a CA adhesive composition, showing improved retention of toughness after heat ageing of cured bonds, which comprises a) a toughener (e.g., a core-shell copolymer such as an MBS, ABS, or MABS copolymer), and b) CA-compatible, toughener-compatible sustainer (e.g., an organic compound containing one or more unsubstituted or substituted aryl groups, such as diphenylmethane or dichlorobenzene).

In European patent application EP-A-05944317 it is disclosed a CA monomer adhesive formulation which has improved thermal properties resulting from the inclusion in the formulation of an effective amount a mono, poly or hetero aromatic compound having at least three substitutions on an aromatic ring thereof, two or more of said substitutions being specific electron withdrawing groups, one or more of said substitutions being specific leaving groups.

In European patent application EP-A-0764148 it is disclosed that the admixture of bis-cyanoacrylates to an adhesive composition increases the thermal resistance thereof. A CA adhesive comprising 5 wt% and 10 wt% of a bis-cyanoacrylate shows an improvement of the thermal resistance.

More complex technical solutions have been proposed to improve thermal and/or humidity resistance of CA adhesives.

For example, in international patent application WO-A-2017/077091 it is disclosed a CA composition that include a CA component, a rubber toughening component, a component containing at least two (meth)acrylate functional groups and an anhydride component.

Further, in international patent application WO-A-2017/202698 it is disclosed a two-part curable CA-containing composition, which when cured provides improved humidity and thermal resistance, which comprises:
a) a first part comprising a CA composition comprising: a CA component, and a rubber toughening agent comprised of (i) reaction products of the combination of ethylene, methyl acrylate and monomers having carboxylic acid cure sites, (ii) polymers of ethylene and methyl acrylate, and combinations of (i) and (ii); and
b) a second part comprising: a specific 2-substituted benzothiazole or derivative thereof; and where at least one of the first or second part further comprises a component containing at least two (meth)acrylate functional groups, at least a specific benzonitrile compound, and at least one anhydride component.

In international patent application WO-A-2018/202384 it is disclosed a CA composition demonstrating improved thermal ageing performance, including improved tensile strength performance after heat ageing in high humidity. Said composition comprising: a) a CA component; b) a toughening agent comprising a copolymer of polyethylene and polyvinyl acetate; c) a specific component having at least two (meth)acrylate functional groups; d) a benzonitrile component; and e) an anhydride component.

Thus, there is still a need to provide an adhesive that combines improved thermal bonding performance and chemical/hydrolysis resistance.

### Subject-matter of the invention

The subject-matter of the present invention is a cyanoacrylate adhesive composition.

Another aspect of the invention is a syringe or a cartridge comprising the CA adhesive composition.

Another aspect of the invention relates to the use of said cyanoacrylate adhesive composition for bonding substrates.

Another aspect of the invention is a method for bonding substrates using said cyanoacrylate adhesive composition.

Another aspect of the invention is said cyanoacrylate adhesive composition for use in human or animal tissue bonding.

### Detailed description of the invention

The object of the present invention is a cyanoacrylate adhesive composition comprising a monomer composition comprising:
a) between 90 wt% and 99 wt% of cyanoacrylate monomer,
b) between 0.1 wt% and 1.5 wt% of multicyanoacrylate monomer, preferably a bis-cyanoacrylate monomer,
c) between 0.1 wt% and 5 wt% of unsaturated carboxylic acid anhydride,
wherein the wt% is based on the total weight of the monomer composition.

The inventors of the present invention have developed a monomer composition, which may be incorporated to one-component and to two-component cyanoacrylate (CA) adhesives, either photocurable or not, and which shows surprisingly a synergistic effect regarding improvement of thermal and/or chemical/hydrolytic resistance of the cured adhesive. The composition of the invention further provides unexpected advantages as tissue adhesive, which have demonstrated greater duration on the skin as wound sealant than other commercially available tissue adhesives.

Throughout the present description and in the claims, the expressions in singular preceded by the articles "a" or "the" are understood to also include, in a broad manner, the reference to the plural, unless the context clearly indicates the contrary.

In the context of the present invention, it is understood that the term "about" referred to a determined value indicates that a certain variation for said value is accepted, generally of +/- 5 %.

The ranges disclosed in this description include both the lower and the upper limit thereof.

### Monomer composition

The CA adhesive composition comprises a combination of monomers: cyanoacrylate, multicyanoacrylate and unsaturated carboxylic acid anhydride in specific amounts.

Said monomer combination is suitable to be used in a variety of CA adhesive compositions, either one-component, or two-component, either photocurable or non-photocurable.

In an embodiment, the monomer composition comprises:
a) between 96.4 wt% and 99 wt% of cyanoacrylate monomer,
b) between 0.5 wt% and 0.8 wt% of multicyanoacrylate monomer, preferably a bis-cyanoacrylate monomer,
c) between 0.5 wt% and 2.8 wt% of unsaturated carboxylic acid anhydride wherein the wt% is based on the total weight of the monomer composition.

In an embodiment, the monomer composition consists essentially of:
a) between 90 wt% and 99 wt% of cyanoacrylate monomer,
b) between 0.1 wt% and 1.5 wt% of multicyanoacrylate monomer, preferably a bis-cyanoacrylate monomer,
c) between 0.1 wt% and 5 wt% of unsaturated carboxylic acid anhydride wherein the wt% is based on the total weight of the monomer composition.

In an embodiment, the monomer composition consists essentially of:
a) between 96.4 wt% and 99 wt% of cyanoacrylate monomer,
b) between 0.5 wt% and 0.8 wt% of multicyanoacrylate monomer, preferably a bis-cyanoacrylate monomer,
c) between 0.5 wt% and 2.8 wt% of unsaturated carboxylic acid anhydride wherein the wt% is based on the total weight of the monomer composition.

In an embodiment, the monomer composition consists of:
a) between 90 wt% and 99 wt% of cyanoacrylate monomer,
b) between 0.1 wt% and 1.5 wt% of multicyanoacrylate monomer, preferably a bis-cyanoacrylate monomer,
c) between 0.1 wt% and 5 wt% of unsaturated carboxylic acid anhydride wherein the wt% is based on the total weight of the monomer composition.

In an embodiment, the monomer composition consists of:
a) between 96.4 wt% and 99 wt% of cyanoacrylate monomer,
b) between 0.5 wt% and 0.8 wt% of multicyanoacrylate monomer, preferably a bis-cyanoacrylate monomer,
c) between 0.5 wt% and 2.8 wt% of unsaturated carboxylic acid anhydride wherein the wt% is based on the total weight of the monomer composition.

The cyanoacrylate monomer wt%, multicyanoacrylate monomer wt% and unsaturated carboxylic acid anhydride wt% in the monomer composition of the invention are adjusted so that their sum is 100 wt%.

### Cyanoacrylate (CA) monomer

The monomer composition of the CA composition of the invention comprises a CA monomer, preferably selected from 2-methoxyethyl 2-cyanoacrylate, 2-ethoxyethyl 2-cyanoacrylate, ethyl 2-cyanoacrylate, *n*-propyl 2-cyanoacrylate, iso-propyl 2-cyanoacrylate, *n*-butyl 2-cyanoacrylate, *sec*-butyl 2-cyanoacrylate, iso-butyl 2-cyanoacrylate, *tert*-butyl 2-cyanoacrylate, *n*-pentyl 2-cyanoacrylate, neopentyl 2-cyanoacrylate, 1-ethylpropyl 2-cyanoacrylate, 1-methylbutyl 2-cyanoacrylate, *n*-hexyl 2-cyanoacrylate, *n*-heptyl 2-cyanoacrylate, *n*-octyl 2-cyanoacrylate, *2*-octyl 2-cyanoacrylate, 2-ethylhexyl 2-cyanoacrylate, tetrahydrofurfuryl 2-cyanoacrylate, and mixtures thereof.

Preferably, the CA monomer is selected from ethyl 2-cyanoacrylate, 2-methoxyethyl 2-cyanoacrylate, *n*-butyl 2-cyanoacrylate, *n*-hexyl 2-cyanoacrylate, 2-ethylhexyl 2-cyanoacrylate, *2*-octyl 2-cyanoacrylate and mixtures thereof.

In an embodiment, the CA monomer is ethyl 2-cyanoacrylate. In an embodiment, the CA monomer is 2-methoxyethyl 2-cyanoacrylate. In an embodiment, the CA monomer is *n*-butyl 2-cyanoacrylate. In an embodiment, the CA monomer is *n-*hexyl 2-cyanoacrylate. In an embodiment, the CA monomer is 2-octyl 2-cyanoacrylate. In an embodiment, the CA monomer is 2-ethylhexyl 2-cyanoacrylate.

Such monomers can be prepared by methods known by the skilled in the art such as the method described, for example, in US2467927. Some of them, such as, for example, ethyl 2-cyanoacrylate (ECA), *n*-butyl 2-cyanoacrylate (BCA), *n*-hexyl 2-cyanoacrylate (HCA), *2*-octyl 2-cyanoacrylate (OCA) and 2-methoxyethyl 2-cyanoacrylate (MECA) are commercially available.

The content of CA monomer is comprised between 90 wt% and 99 wt%, preferably between 92 wt% and 99 wt%, more preferably between 94 wt% and 99 wt%, and yet more preferably between 96.4 wt% and 99 wt%, wherein the wt% is based on the total weight of the monomer composition.

### Multicyanoacrylate monomer

The monomer composition of the CA composition of the invention comprises a multicyanoacrylate monomer, preferably a bis-cyanoacrylate monomer.

A multicyanoacrylate monomer includes at least two 2-cyanoacrylate groups in the molecule, and it is also designated as poly(2-cyanoacrylate) monomer. Examples of multicyanoacrylate monomers are tetra-cyanoacrylate monomers, tri-cyanoacrylate monomers, and bis-cyanoacrylate monomers, such as, for example, pentaerythritol tetra-cyanoacrylate, glyceryl tri-cyanoacrylate, trimethylolpropane tri-cyanoacrylate.

In an embodiment, the multicyanoacrylate monomer includes further acrylate functional groups, such as acrylate-cyanoacrylate monomers disclosed in US-A-2020/0048513.

A bis-cyanoacrylate monomer includes two 2-cyanoacrylate groups in the molecule, and it is also designated as bis(2-cyanoacrylate) monomer.

The multicyanoacrylate monomer is included in the composition of the invention as a crosslinking agent with other monofunctional monomers, for example CA monomers or acrylate monomers.

Tri- and tetracyanoacrylate monomers may be prepared according to a process already disclosed in prior art, such as, for example, in US-A-2020/0048513.

Bis-cyanoacrylates may be prepared according to a process already disclosed in prior art, such as, for example, in US4012402 or US6096848.

In a preferred embodiment the multicyanoacrylate monomer is a bis-cyanoacrylate monomer.

The bis-cyanoacrylate suitable to be included in the CA composition of the invention may be any compound including two 2-cyanoacrylate groups, such as, for example: methylene glycol bis(2-cyanoacrylate), ethylene glycol bis(2-cyanoacrylate), 1,3-propanediol bis(2-cyanoacrylate), 1,3-butanediol bis(2-cyanoacrylate), 1,4-butanediol bis(2-cyanoacrylate), *trans*-2-Butene-1,4-diol bis(2-cyanoacrylate), 1,4-cyclohexanedimethanol bis(2-cyanoacrylate), 1,5-pentanediol bis(2-cyanoacrylate), 1,6-hexanediol bis(2-cyanoacrylate), 2,5-hexanediol bis(2-cyanoacrylate), 1,7-heptanediol bis(2-cyanoacrylate), 1,8-octanediol bis(2-cyanoacrylate), 1,9-nonanediol bis(2-cyanoacrylate), 1,10-decanediol bis(2-cyanoacrylate), 1, 12-dodecanediol bis(2-cyanoacrylate), 2-butyne-1,4-diol bis(2-cyanoacrylate), neopentyl glycol bis(2-cyanoacrylate), 1,4-bis(hydroxymethyl) benzene bis(2-cyanoacrylate), 1,3-bis(hydroxymethyl) tetramethyl-disiloxane bis(2-cyanoacrylate), or bis(4-hydroxybutyl) ether bis(2-cyanoacrylate).

In an embodiment, the bis-cyanoacrylate is 1,6-hexanediol bis(2-cyanoacrylate).

The content of multicyanoacrylate monomer, preferably, bis-cyanoacrylate monomer, is comprised between 0.1 wt% and 1.5 wt%, preferably between 0.2 wt% and 1.3 wt%, more preferably between 0.3 wt% and 1.0 wt%, and yet more preferably between 0.5 wt% and 0.8 wt%, wherein the wt% is based on the total weight of the monomer composition.

In a preferred embodiment, the content of multicyanoacrylate monomer, preferably, bis-cyanoacrylate monomer, is 0.6 wt%, wherein the wt% is based on the total weight of the monomer composition.

### Unsaturated carboxylic acid anhydride

The monomer composition of the CA composition of the invention comprises an unsaturated carboxylic acid anhydride.

In the scope of the invention, the term "unsaturated carboxylic acid anhydride" includes also aromatic acid anhydrides.

A carboxylic acid anhydride is a compound that results when two carboxylic acids combine and lose water. Said compound has two acyl groups bonded to the same oxygen atom. A generic example of carboxylic acid anhydride may be represented by R¹-C(O)-O-(O)C-R², wherein R¹ and R² may be the same (symmetrical anhydrides) or different (unsymmetrical anhydrides), and may be linked forming a saturated or unsaturated ring.

In the composition of the invention, unsaturated carboxylic acid anhydrides suitable to be included in the monomer composition are, for example, maleic anhydride, itaconic anhydride, phthalic anhydride, isophthalic anhydride, and mixtures thereof.

In an embodiment, one or more of the hydrogen atoms of the unsaturated carboxylic acid is replaced by groups such as halogen (e.g., chloro, bromo or iodo radical), C₁-C₈ alkyl groups, preferably C₁-C4 alkyl groups, C₁-C₈ alkenyl groups, preferably C₁-C4 alkenyl groups, C₃-C₈ cycloalkyl groups, preferably C₃-C₆ cycloalkyl groups, or C₄-C₈ cycloalkenyl groups, preferably C₄-C₆ cycloalkenyl groups.

In a preferred embodiment, the unsaturated carboxylic acid anhydride is selected from maleic anhydride, phthalic anhydride and mixtures thereof.

In an embodiment, the unsaturated carboxylic anhydride is maleic anhydride.

In an embodiment, the unsaturated carboxylic anhydride is phthalic anhydride.

In an embodiment, the unsaturated carboxylic anhydride is a combination of maleic anhydride and phthalic anhydride.

The content of the unsaturated carboxylic acid anhydride is comprised between 0.1 wt% and 5 wt%, preferably between 0.2 wt% and 4 wt%, more preferably between 0.3 wt% and 3 wt%, and yet more preferably between 0.5 wt% and 2.8 wt%, wherein the wt% is based on the total weight of the monomer composition.

In an embodiment, the content of the unsaturated carboxylic acid anhydride is comprised between 0.5 wt% and 2.2 wt%, preferably between 1.0 wt% and 2.2 wt%, wherein the wt% is based on the total weight of the monomer composition.

In a more preferred embodiment, the unsaturated carboxylic anhydride is a mixture of maleic anhydride and phthalic anhydride, wherein the content of maleic anhydride is between 0.05 wt% and 0.5 wt% and the content of phthalic anhydride is between 1.0 wt% and 2.0 wt%, wherein the wt% is based on the total weight of the monomer composition.

In an embodiment, the content of bis-cyanoacrylate, preferably 1,6-hexanediol bis(2-cyanoacrylate). is 0.6 wt% and the unsaturated carboxylic acid anhydride is a combination of maleic anhydride and phthalic anhydride, wherein the content of maleic anhydride is comprised between 0.05 wt% and 0.5 wt% and the content of phthalic anhydride is between 1.0 wt% and 2.0 wt%, wherein the wt% is based on the total weight of the monomer composition.

### Cyanoacrylate adhesive composition

As exposed above, CA adhesives are well known in the art, as disclosed, for example, in S. Ebnesajjad, Ed., Handbook of adhesives and surface preparation, Elsevier, London, 2011, section 8.12.

In many industrial and domestic applications CAs are used in form of one-component (1K CA), as they polymerize rapidly when they are in the form of a thin film between two substrates in the presence of anions or nucleophilic species.

For other applications CAs are used in form of two-component (2K CA), wherein one component contains the CA monomer and the other component contains a composition comprising plasticizer and initiator, or a non-CA polymerisable monomer mixture and an initiator.

Two-component CA adhesive compositions are usually mixed in a volumetric ratio between the CA-component and the initiator-component of 1:1, 2:1, 4:1, 6:1, 8:1, 10:1, 20:1 or 50:1. In a preferred embodiment, the ratio is selected from 4:1 and 10:1. In one embodiment, the non-photocurable 2K CA adhesive composition has a ratio of 4:1. In one embodiment, the photocurable 2K CA adhesive composition has a ratio of 10:1.

CA adhesives are non-photocurable or photocurable depending on the composition.

Non-photocurable one- or two-component CA adhesive compositions may comprise further components in addition to the monomers, such as, radical polymerization inhibitors and acid stabilizing agents to prevent an earlier polymerization of the monomers. Further additives, which may be present in said composition are thickening agents, plasticizers, thixotropic agents, crosslinking agents, accelerating agents, and mixtures thereof.

Photocurable one- or two-component CA adhesive compositions comprise a photoinitiator system, wherein an additional curing mechanism enables full and fast cure of any excess adhesive and effectively seals and strengthens the joined parts by using visible or UV light. Said photoinitiator system include at least one photoinitiator, optionally combined with and adjuvant, such as ferrocene, or derivatives thereof.

In two-component CA adhesive compositions an initiator is present in part B of the composition.

Certain CA compositions with an additional light cure capability have been described in prior art, for example, in international patent application WO-A-98/38260.

The CA adhesive composition of the invention may be one-component (1K CA) or two-component (2K CA).

In one embodiment, the CA adhesive composition is one-component.

In one embodiment, the CA adhesive composition is two-component.

In one embodiment, the CA adhesive composition is non-photocurable one-component.

In one embodiment, the CA adhesive composition is photocurable one-component.

In one embodiment, the CA adhesive composition is non-photocurable two-component.

In one embodiment, the CA adhesive composition is photocurable two-component.

In a preferred embodiment, the CA composition is non-photocurable one-component, wherein the content CA monomer is comprised between 96.4 wt% and 99 wt%, the content of multicyanoacrylate monomer, preferably 1,6-hexanediol bis(2-cyanoacrylate), is comprised between 0.5 wt% and 0.8 wt%, the content of maleic anhydride is comprised between 0.05 wt% and 0.5 wt% and the content of phthalic anhydride is between 1.0 wt% and 2.0 wt%, wherein the wt% is based on the total weight of the monomer composition.

In a preferred embodiment, the CA composition is photocurable one-component, wherein the content CA monomer is comprised between 96.4 wt% and 99 wt%, the content of multicyanoacrylate monomer, preferably 1,6-hexanediol bis(2-cyanoacrylate) is comprised between 0.5 wt% and 0.8 wt%, the content of maleic anhydride is comprised between 0.05 wt% and 0.5 wt% and the content of phthalic anhydride is between 1.0 wt% and 2.0 wt%, wherein the wt% is based on the total weight of the monomer composition.

In a preferred embodiment, the CA composition is non-photocurable two-component, wherein the content CA monomer is 96.4 wt%, the content of multicyanoacrylate monomer, preferably 1,6-hexanediol bis(2-cyanoacrylate), is 0.8 wt%, and the content of the unsaturated carboxylic acid anhydride, preferably anhydride maleic, is 2.8 wt%, wherein the wt% is based on the total weight of the monomer composition.

In a preferred embodiment, the CA composition is photocurable two-component, wherein the content CA monomer is 96.4 wt%, the content of multicyanoacrylate monomer, preferably 1,6-hexanediol bis(2-cyanoacrylate), is 0.8 wt%, and the content of the unsaturated carboxylic acid anhydride, preferably anhydride maleic, is 2.8 wt%, wherein the wt% is based on the total weight of the monomer composition.

### Radical polymerisation inhibitor

The CA adhesive composition of the invention comprises radical polymerisation inhibitors to prevent premature polymerisation due to any radical mechanism such as autoxidation or thermal polymerisation

Radical polymerisation inhibitors are preferably selected from 4-methoxyphenol, hydroquinone, hydroquinone monomethyl ether, hydroxytoluene butyl ether, hydroxyanisole butyl ether, 2,2'-methylenebis(4-methyl-6-*tert*-butylphenol), 4,4'-methylenbis(2,6-di-*tert*-butylphenol) and mixtures thereof; more preferably selected from the group consisting of hydroxyanisole butyl ether, 2,2'-methylenebis(4-methyl-6-*tert-*butylphenol), hydroxytoluene butyl ether, and mixtures thereof.

The composition comprises between 0.05 wt% and 1.5 wt%, preferably between 0.08 wt% and 0.6 wt%, more preferably between 0.09 wt% and 0.5 wt%, and yet more preferably between 0.1 wt% and 0.4 wt% of a radical polymerisation inhibitor, wherein the wt% is based on the total weight of the composition.

Radical polymerisation inhibitors are commercially available through companies, such as, for example, TCI or Sigma-Aldrich.

### Acid stabilizing agents

In an embodiment, the CA adhesive composition further comprises an acid stabilizing agent.

Acid stabilizing agents are inhibitors of the anionic polymerisation and may be selected from the group consisting of Bronsted acids, Lewis acids, and mixtures thereof.

The acid stabilizing agent may be selected from boron trifluoride, boron trifluoride etherate, boron trifluoride dihydrate, trimethylsilyl triflate, sulphur dioxide, methanesulfonic acid, and mixtures thereof. In a more preferred embodiment, the acid stabilizing agent is selected from sulphur dioxide, boron trifluoride etherate, methanesulfonic acid, and mixtures thereof.

In an embodiment, the composition further comprises a combination of boron trifluoride etherate and methanesulfonic acid.

In an embodiment, the composition further comprises a combination of sulphur dioxide and boron trifluoride etherate.

In an embodiment, the composition further comprises a combination of sulphur dioxide, boron trifluoride etherate and methanesulfonic acid.

The content of sulphur dioxide is usually comprised between 0.0005 wt% and 0.02 wt%, preferably between 0.0006 wt% and 0.015 wt%, more preferably between 0.00065 wt% and 0.01 wt%, wherein the wt% is based on the total weight of the composition.

The content of boron trifluoride etherate is usually comprised between 0 and 0.004 wt%, preferably between 0.0001 wt% and 0.003 wt%, more preferably between 0.0005 wt% and 0.0015 wt%, wherein the wt% is based on the total weight of the composition.

The content of methanesulfonic acid is usually comprised between 0 and 0.005 wt%, preferably between 0.0005 wt% and 0.003 wt%, more preferably between 0.00075 wt% and 0.0015 wt%, wherein the wt% is based on the total weight of the composition.

### Photoinitiators

The photocurable one-component CA adhesive composition further contains a photoinitiator.

A photoinitiator is a molecule that creates reactive species (free radicals, cations or anions) when exposed to radiation (UV or visible). Photoinitiators are well known in the state of the art and are usually selected from AIBN, benzoyl peroxide, and derivatives of phosphine oxides. Photochemistry of this type of compounds is disclosed, for example, in Slugget et al., (2,4,6-Trimethylbenzoyl)phosphine Oxide Photochemistry. A Direct Tome-Resolved Spectroscopic of Both Radical Fragments, J. Am. Chem Soc., 1995, 117, 5148-5153, in Meereis et a/., BAPO as an alternative photoinitiator for the radical polymerisation of dental resins, Dental Materials, 2014, 30, 945-953, and in Ikemura et a/., UV-VIS spectra and photoinitiation behaviours of acylphosphine oxide and bisacylphosphine oxide derivatives in unfilled, light-cured dental resins. Dental. Mat. J., 2008, 27(6), 765-774.

In an embodiment, the composition comprises a benzoyl phosphine oxide as photoinitiator. Benzoyl phosphine oxides are preferably selected from phenyl bis(2,4,6-trimethylbenzoyl)phosphine oxide (BAPO), diphenyl (2,4,6-trimethylbenzoyl)phosphine oxide (TPO), benzoyl-diphenylphosphine oxide (BDPO), 2,4,6-trimethylbenzoyl-methoxy-phenylphosphine oxide (TMMPO), phthaloyl-bis(diphenylphosphine oxide) (PBDPO), 2,6-difluoro benzoyl-diphenylphosphine oxide (DFDPO), and mixtures thereof, more preferably selected from phenyl bis(2,4,6-trimethylbenzoyl)phosphine oxide (BAPO), diphenyl (2,4,6-trimethylbenzoyl)phosphine oxide (TPO), and mixtures thereof.

The content of photoinitiator in the composition is comprised between 0.1 wt% and 0.8 wt%, preferably 0.2 wt% and 0.6 wt%, more preferably between 0.24 wt% and 0.44 wt%, and yet more preferably between 0.3 wt% and 0.4 wt% of wherein the wt% is based on the total weight of the CA composition. This range corresponds to between 1000 ppm and 8000 ppm, preferably between 2000 ppm and 6000 ppm, more preferably between 2400 ppm and 4400 ppm, and yet more preferably between 3000 ppm and 4000 ppm.

Benzoyl phosphine oxides are commercially available, or can be prepared as disclosed, for example, in Ikemura *et al., op.cit.* The photoinitiators BAPO and TPO are commercially available through companies, such as, for example, BASF or Rahn.

### Ferrocene

The photocurable one-component CA adhesive composition may contain ferrocene as adjuvant of the photoinitiator.

Ferrrocene is the organometallic compound named bis(*η*⁵-cyclopentadienyl) iron and defined by the formula Fe(C₅H₅)₂ and CAS Registry Number 102-54-5. The molecule consists of two cyclopentadienyl rings bound on opposite sides of a central iron atom. It is commercially available through companies such as, for example, American elements or Sigma-Aldrich.

As disclosed in international patent application WO-A-2017/021785, ferrocene significantly improves the photosensitivity for polymerisation of ionically (anionically or zwitterionically) susceptible CA monomers, by interaction with the photolysis products from free radical photoinitiators.

The content of ferrocene in the photocurable one-component CA adhesive composition is comprised between 0.0055 wt% and 0.0295 wt%, preferably between 0.0075 wt% and 0.0275 wt%, more preferably between 0.015 wt% and 0.025 wt%, and yet more preferably it is 0.02 wt%, wherein the wt% is based on the total weight of the CA composition. This range corresponds to between 55 ppm and 295 ppm of ferrocene, preferably between 75 ppm and 275 ppm, more preferably between 150 ppm and 250 ppm, and yet more preferably it is 200 ppm.

### Initiator

As exposed above the two-component CA compositions contain an initiator in part B of the CA composition, located in a separated container.

The use of xanthines, for example, caffeine, as initiating agent in cyanoacrylate compositions of two components is described in, for example, the U.S. patent US4042442 and also in the Spanish patent application P201331566.

The initiator of the present invention is selected from xanthines, quaternary ammonium salts, metal salts of carboxylic acids, amines, phosphines, and mixtures thereof.

Xanthines are compounds that belong to a chemical group denominated purine bases, which includes among others, guanine, adenine, hypoxanthine, caffeine, theobromine and theophylline. Xanthines selected from the group consisting of caffeine and theobromine are preferably used.

The quaternary ammonium salts can also be used as initiators in the component B, choline chloride is preferably used.

The carboxylic acids are preferably selected from monocarboxylic acids having C₂-C₂₀ linear or branched, saturated or unsaturated chain, preferably acetic acid, 2-ethylhexanoic acid, palmitic acid, and stearic acid; acrylic acid, and methacrylic acid. The metal salts are preferably selected from alkali salts, alkaline earth salts, aluminium salts, and zinc (II) salts, more preferably from lithium, sodium, potassium, calcium, magnesium, barium, aluminium, and zinc salts. Still more preferably, the metal salts are selected from the group consisting of lithium stearate, sodium stearate, potassium stearate, zinc stearate, calcium stearate, magnesium stearate, barium stearate, lithium 2-ethylhexanoate, sodium 2-ethylhexanoate, potassium 2-ethylhexanoate, zinc 2-ethylhexanoate, calcium 2-ethylhexanoate, magnesium 2-ethylhexanoate, barium 2-ethylhexanoate, sodium acetate, calcium acetate, zinc acetate, calcium (meth)acrylate, barium (meth)acrylate, aluminium (meth)acrylate, and zinc (meth)acrylate; more preferably is calcium stearate, barium stearate, calcium acrylate, barium acrylate. The commercial metal salts of stearic acid are generally mixtures of palmitic and stearic acids in different proportions, although the skilled in the art denominates them stearates.

The amines are selected from, for example, aniline, *o*-toluidine, *m-*toluidine, *p*-toluidine, *N*-methylaniline, *N*-ethylaniline, *N*-*n*-propylaniline, *N-*isopropylaniline, *N*-methyl-*o*-toluidine, *N*-methyl-*m*-toluidine, *N*-methyl-*p*-toluidine, *N-*ethyl-*o*-toluidine, *N*-ethyl-*m*-toluidine, *N*-ethyl-*p*-toluidine, *N,N*-dimethylaniline, *N*,*N-*diethylaniline, *N,N*-di-*n*-propylaniline, *N,N*-diisopropylaniline, *N*-methyl-*N*-ethylaniline, *N-*methyl-*N*-*n*-propylaniline, *N*-ethyl-*N-n*-propylaniline, *N,N*-dimethyl-*o*-toluidine, *N,N-*dimethyl-*m*-toluidine, *N,N*-dimethyl-*p*-toluidine, *N,N-*diethyl-*o*-toluidine, *N,N*-diethyl-*m-*toluidine, *N*, *N*-diethyl-*p*-toluidine and *N,N*-dimethyl-3-aminophenol.

In a particularly preferred embodiment, the initiating agent is selected from the group consisting of caffeine, calcium acrylate, barium acrylate, a combination of caffeine and calcium stearate, a combination of caffeine and calcium acrylate, a combination of caffeine and barium stearate, and a combination of caffeine and barium acrylate.

Examples of suitable phosphines include, but are not limited to, phosphines such as aromatic-containing phosphines, for example, phenylphosphine, diphenylphosphine, and triphenyl phosphine; alkyl-containing phosphines such as methyl phosphine, diethyl phosphine, ethyl phosphine, dichlorodiphenyl phosphine, dichlorophenyl phosphine, and dimethyphenyl phosphine. The phosphines can be a salt, include a metal halogen and/or a functional group. Some examples: 2,2'-bis(diphenylphosphino-1,1'-biphenyl; 4,12-bis(diphenylphosphino)-[2.2]-paracyclophane; 4,12-bis[di(3,5-xylyl)phosphino]-[2.2]-paracyclophane; and 1,4-bis(diphenylphosphino)butane.

### Additives

The CA adhesive composition may further contain one or more additives. Additives are selected, for example, from thickening agents, plasticizers, thixotropic agents, crosslinking agents, accelerating agents, and mixtures thereof.

In an embodiment, the composition further comprises a combination of plasticizer and thickening agent.

### Thickening agent

A thickening agent is added to increase the viscosity of a CA-type adhesive composition. A suitable thickener or thickening agent for the composition may be selected from those which are compatible with the CA monomers contained therein.

Polymers can be used as thickeners, such as, for example, poly(meth)acrylates, acylated cellulose polymers, polyvinyl acetates, partially hydrolysed polyvinyl acetates, polyvinylpyrrolidones, vinyl acetate copolymers, copolymers of (meth)acrylates with butadiene and styrene, copolymers of vinyl chloride and acrylonitrile, copolymers of ethylene and vinyl acetate, and mixtures thereof.

Certain fumed silica fillers, optionally treated with polydialkylsiloxanes or trialkoxyalkylsilanes, can also be used as thickening agent.

In the scope of the invention, the thickening agent is selected from poly(meth)acrylates, acylated cellulose polymers, polyvinyl acetates, partially hydrolysed polyvinyl acetates, polyvinylpyrrolidones, vinyl acetate copolymers, copolymers of (meth)acrylates with butadiene and styrene, copolymers of vinyl chloride and acrylonitrile, copolymers of ethylene and vinyl acetate, fumed silica, fumed silica treated with polydialkylsiloxanes or trialkoxyalkylsilanes, and mixtures thereof.

Preferably, in the composition of the invention the thickener is selected from the group consisting of vinyl acetate copolymers, fumed silica, and mixtures thereof.

Generally, the content of thickener is comprised between about 3 wt% and about 20 wt%, preferably between about 5 wt% and about 18 wt%, and more preferably between about 5 wt. % and about 15 wt%, wherein the wt% is based on the total weight of the CA composition.

These thickening agents are well known to the skilled person and have been described in the prior art.

Polymers suitable as thickening agent are commercially available through companies such as, for example, Wacker, Evonik or Lanxess.

Fumed silica is commercially available through companies such as, for example, Evonik.

### Plasticizer

The CA adhesive composition may comprise a plasticizer. Usually, the plasticizer is selected from organic esters such as, for example, acetates, carbonates, citrates, succinates, azelates, adipates, sebacates, stearates, myristates and phthalates.

Preferably the plasticizer is selected from triacetin, propylene carbonate, and mixtures thereof.

Typically, the content of the plasticizer in the composition is comprised between about 1 wt% and about 20 wt. %, preferably between about 3 wt% and about 15 wt. %, more preferably between about 5 wt% and about 12 wt%, and yet more preferably between about 8 wt% and about 11 wt%, wherein the wt% is based on the total weight of the CA composition.

### Thixotropic agent

A thixotropic agent may be added to the CA adhesive composition to manage its rheological behaviour.

Preferably, the thixotropic agent is silica, which is available as fumed silica, optionally hydrophobized, and precipitated silica. In a more preferred embodiment, it is hydrophobized fumed silica. Silica may act both as thickening agent and as thixotropic agent.

Typically, the content of thixotropic agent in the composition is comprised between about 2 wt% and about 10 wt%, preferably between about 3 wt% and about 8 wt%, and more preferably between about 4 wt% and about 7 wt%, wherein the wt% is based on the total weight of the CA composition.

Fumed silica is commercially available through companies such as, for example, Evonik.

### Crosslinking agents

In an embodiment, the photocurable two-component CA adhesive composition may further comprise a tri- or difunctional monomeric crosslinking agent, without having 2-cyanoacrylate groups, to improve the cohesive strength of adhesive bonds formed therefrom.

Such crosslinking agents are known to the skilled person in the art, and are disclosed, for example in US patent US3940362, and commercially available through companies, such as, for example, IGM.

Examples of suitable crosslinking agents include triallyl isocyanurates, alkylene diacrylates, alkylene dimethylacrylates, bisphenol A alkoxylated diacrylate, bisphenol A epoxy diacrylate, trimethylolpropane trimethacrylate and trimethylolpropane triacrylate.

The content of the crosslinking agent is usually comprised between about 0.1 wt% to about 50 wt%, preferably between about 0.5 wt% and about 40 wt%, wherein the wt% is based on the total weight of the CA composition.

### Accelerating agent

The CA adhesive composition may further comprise an accelerating agent.

A suitable accelerating agent or accelerator for the composition is a crown ether.

Typically, the content of the accelerating agent in the composition is comprised between about 0.01 wt% and about 0.8 wt%, preferably between about 0.05 wt% and about 0.5 wt%, and more preferably between about 0.1 wt% and about 0.3 wt%, wherein the wt% is based on the total weight of the CA composition.

In a preferred embodiment, the non-photocurable one-component cyanoacrylate adhesive composition comprises, further to the monomer composition, one or more of the components selected from: radical polymerization inhibitors, acid stabilizing agents, thickening agents, and thixotropic agents.

In a preferred embodiment, the photocurable one-component cyanoacrylate adhesive composition comprises, further to the monomer composition, one or more components selected from radical polymerization inhibitors, acid stabilizing agents, thickening agents, thixotropic agents, photoinitiators, and ferrocene.

In a preferred embodiment, the non-photocurable two-component cyanoacrylate adhesive composition comprises, further to the monomer composition, one or more components selected from radical polymerization inhibitors, acid stabilizing agents, thickening agents, thixotropic agents, initiators, and plasticizers.

In a preferred embodiment, the photocurable two-component cyanoacrylate adhesive composition comprises, further to the monomer composition, one or more components selected from radical polymerization inhibitors, acid stabilizing agents, thickening agents, thixotropic agents, initiators, plasticizers, photoinitiators, and ferrocene.

### Syringe or cartridge

The CA adhesive composition may be dispensed from packages, such as a syringe or from reservoir pots.

Also, part of the object of the invention is a syringe or a cartridge comprising the CA adhesive composition.

### Use of the cyanoacrylate adhesive composition

The use of the CA adhesive composition for bonding substrates also forms part of the subject-matter of the invention.

As shown in the examples, the compositions of the invention show a synergistic effect regarding the loss of adhesion comparing the shear strength, expressed in MPa, determined after 24 h at room temperature with the shear strength determiner after further 24 h at 120 °C. The compositions of the invention comprising CA monomer, multicyanoacrylate monomer, preferably bis-cyanoacrylate monomer, and unsaturated carboxylic acid anhydride show a significant lower loss of adhesion in comparison to the compositions that do not have either the multicyanoacrylate monomer component or the anhydride component.

Resistance of the adhesive obtained by curing the CA adhesive composition of the invention was superior to a commercially available CA composition after ageing at elevated temperature.

A method for bonding substrates also forms part of the subject-matter of the invention. Said method for bonding substrates comprises the steps of:
1) applying the cyanoacrylate adhesive composition of the invention to at least one of the substrates,
2) placing together the substrates,
3) optionally irradiating with a light of a wavelength comprised between 380 nm and 415 nm, and
4) maintaining the substrates clamped up to fixation.

In case of a photocurable CA adhesive composition, the source of radiation emitting electromagnetic waves may be selected from ultraviolet light, visible light, and a combination thereof.

As light source it may be employed LED irradiating in a wavelength comprised between 380 nm and 415 nm, having a potency comprised between 0.4 W and 36 W.

In an embodiment, the composition is for use as tissue adhesive (bioadhesive) and biosealant, preferably in human or animal tissue bonding. In a preferred embodiment, the composition is applied topically. In a preferred embodiment, the composition is applied internally in procedures selected from the group consisting of cardiovascular, peripheral-vascular, orthopaedic, cardio-thoracic, gynaecological, neuro and general abdominal surgery.

A tissue adhesive or biosealant is a liquid composition that undergo a polymerization reaction upon exposure to a moist surface, such as skin, optionally buy means of light, changing to a polymer that forms a strong tissue bond.

Additionally, the claimed composition allows the preparation of adhesives with optimal heat release, adequate viscosities and thixotropy which cover a wide range of viscosities presenting excellent physical and mechanical properties to be used as adhesive and sealants for industrial, consumer, medical and cosmetic applications in which controlled exotherm could be of interest.

Compositions of the invention provide unexpected advantages as tissue adhesive, which have demonstrated longer duration on the skin as wound sealant.

The combination of multicyanoacrylate and unsaturated carboxylic acid anhydride enables the preparation of CA adhesive compositions, which after curing show an improved thermal bonding performance and chemical/hydrolysis resistance

The invention comprises the following embodiments:
1.- A cyanoacrylate adhesive composition, characterized in that it comprises a monomer composition comprising:
   a) between 90 wt% and 99 wt%, preferably between 92 wt% and 99 wt%, more preferably between 94 wt% and 99 wt%, and yet more preferably between 96.4 wt% and 99 wt% of cyanoacrylate monomer,
   b) between 0.1 wt% and 1.5 wt%, preferably between 0.2 wt% and 1.3 wt%, more preferably between 0.3 wt% and 1.0 wt%, and yet more preferably between 0.5 wt% and 0.8 wt% of multicyanoacrylate monomer, preferably a bis-cyanoacrylate monomer,
   c) between 0.1 wt% and 5 wt%, preferably between 0.2 wt% and 4 wt%, more preferably between 0.3 wt% and 3 wt%, and yet more preferably between 0.5 wt% and 2.8 wt% of unsaturated carboxylic acid anhydride,
   wherein the wt% is based on the total weight of the monomer composition.
2.- The cyanoacrylate adhesive composition according to embodiment 1, characterized in that it comprises a monomer composition comprising:
   a) between 96.4 wt% and 99 wt% of cyanoacrylate monomer,
   b) between 0.5 wt% and 0.8 wt% of multicyanoacrylate monomer, preferably a bis-cyanoacrylate monomer,
   c) between 0.5 wt% and 2.8 wt% of unsaturated carboxylic acid anhydride
   wherein the wt% is based on the total weight of the monomer composition.
3.- The cyanoacrylate adhesive composition according to embodiments 1 or 2, characterized in that the cyanoacrylate monomer is selected from 2-methoxyethyl 2-cyanoacrylate, 2-ethoxyethyl 2-cyanoacrylate, ethyl 2-cyanoacrylate, *n*-propyl 2-cyanoacrylate, iso-propyl 2-cyanoacrylate, *n*-butyl 2-cyanoacrylate, *sec*-butyl 2-cyanoacrylate, iso-butyl 2-cyanoacrylate, *tert*-butyl 2-cyanoacrylate, *n*-pentyl 2-cyanoacrylate, neopentyl 2-cyanoacrylate, 1-ethylpropyl 2-cyanoacrylate, 1-methylbutyl 2-cyanoacrylate, *n*-hexyl 2-cyanoacrylate, *n*-heptyl 2-cyanoacrylate, *n*-octyl 2-cyanoacrylate, 2-octyl 2-cyanoacrylate, 2-ethylhexyl 2-cyanoacrylate, tetrahydrofurfuryl 2-cyanoacrylate, and mixtures thereof, preferably it is selected from the group consisting of ethyl 2-cyanoacrylate, 2-methoxyethyl 2-cyanoacrylate, *n*-butyl 2-cyanoacrylate, *n-*hexyl 2-cyanoacrylate, 2-ethylhexyl 2-cyanoacrylate, 2-octyl 2-cyanoacrylate and mixtures thereof.
4.- The cyanoacrylate adhesive composition according to any of embodiments 1 to 3, characterized in that the multicyanoacrylate is a bis-cyanoacrylate monomer.
5.- The cyanoacrylate adhesive composition according to embodiment 4, characterized in that the bis-cyanoacrylate monomer is selected from methylene glycol bis(2-cyanoacrylate), ethylene glycol bis(2-cyanoacrylate), 1,3-propanediol bis(2-cyanoacrylate), 1,3-butanediol bis(2-cyanoacrylate), 1,4-butanediol bis(2-cyanoacrylate), *trans*-2-Butene-1,4-diol bis(2-cyanoacrylate), 1,4-cyclohexanedimethanol bis(2-cyanoacrylate), 1,5-pentanediol bis(2-cyanoacrylate), 1,6-hexanediol bis(2-cyanoacrylate), 2,5-hexanediol bis(2-cyanoacrylate), 1,7-heptanediol bis(2-cyanoacrylate), 1,8-octanediol bis(2-cyanoacrylate), 1,9-nonanediol bis(2-cyanoacrylate), 1,10-decanediol bis(2-cyanoacrylate),1,12-dodecanediol bis(2-cyanoacrylate), 2-butyne-1,4-diol bis(2-cyanoacrylate), neopentyl glycol bis(2-cyanoacrylate), 1,4-bis(hydroxymethyl) benzene bis(2-cyanoacrylate), 1,3-bis(hydroxymethyl) tetramethyl-disiloxane bis(2-cyanoacrylate), or bis(4-hydroxybutyl) ether bis(2-cyanoacrylate), preferably it is 1,6-hexanediol bis(2-cyanoacrylate).
6.- The cyanoacrylate adhesive composition according to any of embodiments 1 to 5, characterized in that the unsaturated carboxylic acid anhydride is selected from maleic anhydride, itaconic anhydride, phthalic anhydride, isophthalic anhydride, and mixtures thereof.
7.- The cyanoacrylate adhesive composition according to embodiment 6, characterized in that the unsaturated carboxylic acid anhydride is selected from maleic anhydride, phthalic anhydride and mixtures thereof.
8.- The cyanoacrylate adhesive composition according to embodiment 7, characterized in that the unsaturated carboxylic acid anhydride is a mixture of maleic anhydride and phthalic anhydride.
9.- The cyanoacrylate adhesive composition according to embodiment 8, characterized in that the content of maleic anhydride is between 0.05 wt% and 0.5 wt% and the content of phthalic anhydride is between 1.0 wt% and 2.0 wt%, wherein the wt% is based on the total weight of the monomer composition.
10.- The cyanoacrylate adhesive composition according to embodiment 1, characterized in that the content of multicyanoacrylate monomer, preferably 1,6-hexanediol bis(2-cyanoacrylate), is 0.6 wt% and the unsaturated carboxylic acid anhydride is a combination of maleic anhydride and phthalic anhydride, wherein the content of maleic anhydride is comprised between 0.05 wt% and 0.5 wt% and the content of phthalic anhydride is between 1.0 wt% and 2.0 wt%, wherein the wt% is based on the total weight of the monomer composition.
11.- The cyanoacrylate adhesive composition according to any of embodiments 1 to 10, characterized in that the cyanoacrylate composition is either one-component or two-component.
12.- The cyanoacrylate adhesive composition according to any of embodiments 1 to 11, characterized in that the cyanoacrylate composition is either non-photocurable or photocurable.
13.- The cyanoacrylate adhesive composition according to any of embodiments 1 to 12, characterized in that the cyanoacrylate composition is non-photocurable one-component.
14.- The cyanoacrylate adhesive composition according to any of embodiments 1 to 12, characterized in that the cyanoacrylate composition is photocurable one-component.
15.- The cyanoacrylate adhesive composition according to any of embodiments 1 to 12, characterized in that the cyanoacrylate composition is non-photocurable two-component.
16.- The cyanoacrylate adhesive composition according to any of embodiments 1 to 12, characterized in that the cyanoacrylate composition is photocurable two-component.
17.- The cyanoacrylate adhesive composition according to embodiment 1, characterized in that the cyanoacrylate composition is non-photocurable one-component, wherein the content of cyanoacrylate monomer is comprised between 96.4 wt% and 99 wt%, the content of multicyanoacrylate monomer, preferably 1,6-hexanediol bis(2-cyanoacrylate), is comprised between 0.5 wt% and 0.8 wt%, the content of maleic anhydride is comprised between 0.05 wt% and 0.5 wt% and the content of phthalic anhydride is between 1.0 wt% and 2.0 wt%, wherein the wt% is based on the total weight of the monomer composition.
18.- The cyanoacrylate adhesive composition according to embodiment 1, characterized in that the cyanoacrylate composition is photocurable one-component, wherein the content of cyanoacrylate monomer is comprised between 96.4 wt% and 99 wt%, the content of multicyanoacrylate monomer, preferably 1,6-hexanediol bis(2-cyanoacrylate), is comprised between 0.5 wt% and 0.8 wt%, the content of maleic anhydride is comprised between 0.05 wt% and 0.5 wt% and the content of phthalic anhydride is between 1.0 wt% and 2.0 wt%, wherein the wt% is based on the total weight of the monomer composition.
19.- The cyanoacrylate adhesive composition according to embodiment 1, characterized in that it is non-photocurable two-component, wherein the content of the cyanoacrylate monomer is 96.4 wt%, the content of multicyanoacrylate monomer, preferably 1,6-hexanediol bis(2-cyanoacrylate), is 0.8 wt%, and the content of the unsaturated carboxylic acid anhydride, preferably anhydride maleic, is 2.8 wt%, wherein the wt% is based on the total weight of the monomer composition.
20.- The cyanoacrylate adhesive composition according to embodiment 1, characterized in that it is photocurable two-component, wherein the content of the cyanoacrylate monomer is 96.4 wt%, the content of multicyanoacrylate monomer, preferably 1,6-hexanediol bis(2-cyanoacrylate), is 0.8 wt%, and the content of the unsaturated carboxylic acid anhydride, preferably anhydride maleic, is 2.8 wt%, wherein the wt% is based on the total weight of the monomer composition.
21.- The cyanoacrylate adhesive composition according to any of embodiments 1 to 20, characterized in that it further comprises radical polymerization inhibitors and acid stabilizing agents.
22.- The cyanoacrylate adhesive composition according to any of embodiments 1 to 21, characterized in that it further comprises additives selected from thickening agents, plasticizers, thixotropic agents, crosslinking agents, accelerating agents, photoinitiators, ferrocene, initiator, and mixtures thereof.
23.- The cyanoacrylate adhesive composition according to embodiment 21, characterized in that the radical polymerisation inhibitor is selected from 4-methoxyphenol, hydroquinone, hydroquinone monomethyl ether, hydroxytoluene butyl ether, hydroxyanisole butyl ether, 2,2'-methylenebis(4-methyl-6-*tert*-butylphenol), 4,4'-methylenbis(2,6-di-*tert*-butylphenol) and mixtures thereof, preferably selected from the group consisting of hydroxyanisole butyl ether, 2,2'-methylenebis(4-methyl-6-*tert*butylphenol), hydroxytoluene butyl ether, and mixtures thereof.
24.- The cyanoacrylate adhesive composition according to embodiment 21, characterized in that the acid stabilizing agent is selected from boron trifluoride, boron trifluoride etherate, boron trifluoride dihydrate, trimethylsilyl triflate, sulphur dioxide, methanesulfonic acid, and mixtures thereof, preferably selected from sulphur dioxide, boron trifluoride etherate, methanesulfonic acid, and mixtures thereof.
25.- The cyanoacrylate adhesive composition according to embodiment 22, characterized in that the photoinitiator is selected from phenyl bis(2,4,6-trimethylbenzoyl)phosphine oxide (BAPO), diphenyl (2,4,6-trimethylbenzoyl)phosphine oxide (TPO), benzoyl-diphenylphosphine oxide (BDPO), 2,4,6-trimethylbenzoyl-methoxy-phenylphosphine oxide (TMMPO), phthaloyl-bis(diphenylphosphine oxide) (PBDPO), 2,6-difluoro benzoyl-diphenylphosphine oxide (DFDPO), and mixtures thereof, preferably selected from phenyl bis(2,4,6-trimethylbenzoyl)phosphine oxide (BAPO), diphenyl (2,4,6-trimethylbenzoyl)phosphine oxide (TPO), and mixtures thereof.
26.- The cyanoacrylate adhesive composition according to embodiment 25, characterized in that it further comprises ferrocene as adjuvant of the photoinitiator.
27.- The cyanoacrylate adhesive composition according to embodiment 22, characterized in that the initiator is selected from xanthines, quaternary ammonium salts, metal salts of carboxylic acids, amines, phosphines, and mixtures thereof.
28.- The cyanoacrylate adhesive composition according to embodiment 22, characterized in that the thickening agent is selected from poly(meth)acrylates, acylated cellulose polymers, polyvinyl acetates, partially hydrolysed polyvinyl acetates, polyvinylpyrrolidones, vinyl acetate copolymers, copolymers of (meth)acrylates with butadiene and styrene, copolymers of vinyl chloride and acrylonitrile, copolymers of ethylene and vinyl acetate, fumed silica, fumed silica treated with polydialkylsiloxanes or trialkoxyalkylsilanes, and mixtures thereof, preferably selected from the group consisting of vinyl acetate copolymers, fumed silica, and mixtures thereof.
29.- The cyanoacrylate adhesive composition according to embodiment 22, characterized in that the plasticizer is selected from organic esters selected from acetates, carbonates, citrates, succinates, azelates, adipates, sebacates, stearates, myristates and phthalates, preferably it is selected from triacetin, propylene carbonate, and mixtures thereof.
30.- The cyanoacrylate adhesive composition according to embodiment 22, characterized in that the thixotropic agent is silica, preferably it is hydrophobized fumed silica.
31.- The cyanoacrylate adhesive composition according to embodiment 22, characterized in that the accelerating agent is a crown ether.
32.- The cyanoacrylate adhesive composition according to embodiment 13, characterized in that the non-photocurable one-component cyanoacrylate adhesive composition comprises, further to the monomer composition, one or more components selected from radical polymerization inhibitors, acid stabilizing agents, thickening agents, and thixotropic agents.
33.- The cyanoacrylate adhesive composition according to embodiment 14, characterized in that the photocurable one-component cyanoacrylate adhesive composition comprises, further to the monomer composition, one or more components selected from radical polymerization inhibitors, acid stabilizing agents, thickening agents, thixotropic agents, photoinitiators, and ferrocene.
34.- The cyanoacrylate adhesive composition according to embodiment 15, characterized in that the non-photocurable two-component cyanoacrylate adhesive composition comprises, further to the monomer composition, one or more components selected from radical polymerization inhibitors, acid stabilizing agents, thickening agents, thixotropic agents, initiators, and plasticizers.
35.- The cyanoacrylate adhesive composition according to embodiment 16, characterized in that the photocurable two-component cyanoacrylate adhesive composition comprises, further to the monomer composition, one or more components selected from radical polymerization inhibitors, acid stabilizing agents, thickening agents, thixotropic agents, initiators, plasticizers, photoinitiators, and ferrocene.
36.- A syringe or a cartridge comprising the cyanoacrylate adhesive composition of any of embodiments 1 to 35.
37.- The use of the cyanoacrylate adhesive composition of any of embodiments 1 to 35 for bonding substrates.
38.- A method for bonding substrates, characterized in that it comprises the steps of:
   1) applying the cyanoacrylate adhesive composition of any of embodiments 1 to 35 to at least one of the substrates,
   2) placing together the substrates,
   3) optionally irradiating with a light of a wavelength comprised between 380 nm and 415 nm, and
   4) maintaining the substrates clamped up to fixation.
39.- The cyanoacrylate adhesive composition according to any of embodiments 1 to 35 for use as tissue adhesive (bioadhesive) and biosealant.
40.- The cyanoacrylate adhesive composition for use according to embodiment 39 in human or animal tissue bonding.

### Examples

The following methods were used to assess the performance of the CA compositions of the invention.
1) Viscosity
   The following parameters were used by measuring the viscosity:
   - Brookfield DV2T cone/plate viscometer
   - Spindle 51Z
   - Measuring time: 1 min
   - T: 25 °C
   - Rotational speed: the one that corresponds to about 55 % of Torque.
   - 0.5 ml sample
2) Fixture time
   It is the time at which an adhesive bond can support a 3 kg load for 10 seconds. It has been measured in standardised ABS plastic test specimens from Rocholl GmbH, overlapping 250 mm².
3) Polymerization time
   It is the time at which 0.5 ml of adhesive formulation experiments a 30 % viscosity increase upon addition of 0.3 ml of an initiator solution. It provides an indirect measurement of the acid content in the formulation.
3) Adhesion test
   Tensile shear is reported in MPa as an average of three measurements, and results were obtained in lap shear tests performed according to ISO 4587 on mild steel (MS) substrates (100x25x1.5 mm) with bond area of 250 mm².
4) Humidity test
   The evaluation of the humidity resistance was carried out by preparing glass lap-shear bonds as described above, with a bond area of 250 mm², clamping and allowing them to cure for 1 hour at 22 °C. The bonds were then subjected to ageing at elevated temperature submerged in water (60 °C, 1 h). The lap-shear specimens were then submitted manually to a peeling tensile bond strength.

### Example 1: Non-photocurable 1K Cyanoacrylate composition

A non-photocurable 1K CA composition according to the invention was prepared by mixing the components shown in Table I:

**TABLE I**

| Component/ Example | wt% |
|---|---|
| ECA | 87.648 |
| Maleic anhydride | 1 |
| 1,6-Hexanediol bis-cyanoacrylate | 0.5 |
| Acid stabilizer | q.s. |
| Radical polymerization inhibitor | q.s. |
| Accelerating agent | q.s. |
| Rheological agent | q.s. |
| Total | 100 |

Components such as acid stabilizers, radical polymerization inhibitors, accelerating agents, and rheological agents are included in the formulation according to the common general knowledge of the skilled person in the art.

In Example 1, the CA formulation comprises the monomer composition shown in Table II:

**TABLE II**

| Monomer | wt% |
|---|---|
| CA (ECA) | 98.3 |
| bis-CA (1,6-Hexanediol bis-cyanoacrylate) | 0.6 |
| Maleic anhydride | 1.1 |

### Example 2: Photocurable 1K cyanoacrylate composition

A photocurable 1K CA composition according to the invention was prepared by mixing the components shown in Table III:

**TABLE III**

| Component/ Example | wt% |
|---|---|
| ECA | 89.82 |
| Maleic anhydride | 0.1 |
| Phthalic anhydride | 1.5 |
| 1,6-Hexanediol bis-cyanoacrylate | 0.5 |
| Acid stabilizer | q.s. |
| Radical polymerization inhibitor | q.s. |
| Accelerating agent | q.s. |
| Rheological agent | q.s. |
| Photoinitiator system | q.s. |
| Total | 100 |

In Example 2, the CA formulation comprises the monomer composition shown in Table IV:

**TABLE IV**

| Monomer | wt% |
|---|---|
| CA (ECA) | 97.8 |
| bis-CA (1,6-Hexanediol bis-cyanoacrylate) | 0.5 |
| Maleic anhydride | 0.1 |
| Phthalic anhydride | 1.6 |

### Example 3: Non-photocurable 2K Cyanoacrylate composition

Part A of a non-photocurable 2K CA composition according to the invention was prepared by mixing the components shown in Table V:

**TABLE V**

| Component/ Example | wt% |
|---|---|
| ECA | 87.51 |
| Maleic anhydride | 2.50 |
| 1,6-Hexanediol bis-cyanoacrylate | 0.75 |
| Acid stabilizer | q.s. |
| Radical polymerization inhibitor | q.s. |
| Accelerating agent | q.s. |
| Rheological agent | q.s. |
| Total | 100 |

Part B of the 2K CA composition according to the invention was prepared by mixing the components shown in Table VI:

**TABLE VI**

| Component/ Example | wt% |
|---|---|
| Initiator | 0.16 |
| Plasticizer | 94.94 |
| Rheological agent | q.s. |
| Total | 100 |

In Example 3, the CA formulation comprises the monomer composition shown in Table VII:

**TABLE VII**

| Monomer | wt% |
|---|---|
| CA (ECA) | 96.4 |
| bis-CA (1,6-Hexanediol bis-cyanoacrylate) | 0.8 |
| Maleic anhydride | 2.8 |

### Example 4: Photocurable 2K cyanoacrylate composition

Part A of a photocurable 2K CA composition according to the invention was prepared by mixing the components shown in Table VIII:

**TABLE VIII**

| Component/ Example | wt% |
|---|---|
| BCA | 86.30 |
| Maleic anhydride | 2.50 |
| 1,6-Hexanediol bis-cyanoacrylate | 0.75 |
| Acid stabilizer | q.s. |
| Radical polymerization inhibitor | q.s. |
| Photoinitiator system | q.s. |
| Accelerating agent | q.s. |
| Rheological agent | q.s. |
| Total | 100 |

Part B of the photocurable 2K CA composition according to the invention was prepared by mixing the components shown in Table IX:

**TABLE IX**

| Component/ Example | wt% |
|---|---|
| Initiator | 0.06 |
| Plasticizer | 94.94 |
| Rheological agent | 5 |
| Total | 100 |

In Example 3, the CA formulation comprises the monomer composition shown in Table X:

**TABLE X**

| Monomer | wt% |
|---|---|
| CA (ECA) | 96.4 |
| bis-CA (1,6-Hexanediol bis-cyanoacrylate) | 0.8 |
| Maleic anhydride | 2.8 |

### Examples 5-9: Adhesion test of 1K cyanoacrylate compositions

An adhesion test was performed with 1K CA compositions of Examples 5-9 and Comparative Examples 1-6, prepared according to the process disclosed in Example 1, with the monomer composition as shown in Table XI:

**TABLE XI**

| Example/ wt% Monomers | ECA | bis-CA* | Maleic anhydride | Phthalic anhydride | Loss of adhesion (%) |
|---|---|---|---|---|---|
| 1 | 98.3 | 0.6 | 1.1 | 0 | 35 |
| 2** | 97.8 | 0.5 | 0.1 | 1.6 | 24 |
| 5 | 98.1 | 0.8 | 1.1 | 0 | 43 |
| 6 | 96.6 | 0.6 | 2.8 | 0 | 32 |
| 7 | 96.4 | 0.8 | 2.8 | 0 | 48 |
| 8 | 97.6 | 0.6 | 0.1 | 1.7 | 44 |
| 9 | 97.68 | 0.56 | 0.08 | 1.68 | 45 |
| Comparative Example 1 | 100 | 0 | 0 | 0 | 67 |
| Comparative Example 2 | 99.4 | 0.6 | 0 | 0 | 96 |
| Comparative Example 3 | 99.2 | 0.8 | 0 | 0 | 94 |
| Comparative Example 4 | 98.9 | 0 | 1.1 | 0 | 53 |
| Comparative Example 5 | 97.2 | 0 | 2.8 | 0 | 49 |
| Comparative Example 6 | 98.3 | 0 | 0 | 1.7 | 77 |
| Comparative Example 7 | 98.2 | 0 | 0.1 | 1.7 | 65 |

| | | | | | |
|---|---|---|---|---|---|
| * bis-CA = 1,6-hexanediol bis-cyanoacrylate ** Photocurable 1K composition of Example 2 | | | | | |

The amounts of the rest of components were substantially analogous to those of Example 1.

It can be observed that compositions of the invention show a synergistic effect regarding the loss of adhesion comparing the shear strength, expressed in MPa, determined after 24 h at 120 °C with the shear strength determined after 24 h at room temperature. The compositions of the invention comprising CA monomer, multicyanoacrylate monomer, preferably bis-cyanoacrylate monomer, and unsaturated carboxylic acid anhydride show a significant lower loss of adhesion in comparison to the compositions that do not have either the multicyanoacrylate monomer, preferably bis-cyanoacrylate monomer, or the unsaturated acid anhydride.

### Example 8: Humidity test of a 1K cyanoacrylate composition

Humidity resistance was determined for one-component CA adhesive composition of the invention, containing a monomer composition of 87.5 wt% ECA, 0.5% 1,6-hexanediol bis-cyanoacrylate and 1 wt% of maleic anhydride, prepared following substantially the process and the amounts of the rest of components, as disclosed in Example 1, in comparison to a conventional one-component commercially available CA adhesive composition comprising ECA as monomer.

The evaluation of the humidity resistance was carried out by preparing glass lap-shear bonds as described above, with a bond area of 250 mm², clamping and allowing them to cure for 1 hour at 22 °C. The bonds were then subjected to ageing at elevated temperature submerged in water (60 °C, 1 h). The lap-shear specimens were then submitted manually to a peeling tensile bond strength where the 1K commercially available ECA-based adhesive led to an adhesive failure, while the CA adhesive of the invention led to a substrate failure.

## Claims

1. A cyanoacrylate adhesive composition, **characterized in that** it comprises a monomer composition comprising:
a) between 90 wt% and 99 wt%, preferably between 92 wt% and 99 wt%, more preferably between 94 wt% and 99 wt%, and yet more preferably between 96.4 wt% and 99 wt% of cyanoacrylate monomer,
b) between 0.1 wt% and 1.5 wt%, preferably between 0.2 wt% and 1.3 wt%, more preferably between 0.3 wt% and 1.0 wt%, and yet more preferably between 0.5 wt% and 0.8 wt% of multicyanoacrylate monomer, preferably a bis-cyanoacrylate monomer,
c) between 0.1 wt% and 5 wt%, preferably between 0.2 wt% and 4 wt%, more preferably between 0.3 wt% and 3 wt%, and yet more preferably between 0.5 wt% and 2.8 wt% of unsaturated carboxylic acid anhydride,
wherein the wt% is based on the total weight of the monomer composition.

2. The cyanoacrylate adhesive composition according to claim 1, **characterized in that** the cyanoacrylate monomer is selected from 2-methoxyethyl 2-cyanoacrylate, 2-ethoxyethyl 2-cyanoacrylate, ethyl 2-cyanoacrylate, *n*-propyl 2-cyanoacrylate, iso-propyl 2-cyanoacrylate, *n*-butyl 2-cyanoacrylate, *sec*-butyl 2-cyanoacrylate, iso-butyl 2-cyanoacrylate, *tert*-butyl 2-cyanoacrylate, *n*-pentyl 2-cyanoacrylate, neopentyl 2-cyanoacrylate, 1-ethylpropyl 2-cyanoacrylate, 1-methylbutyl 2-cyanoacrylate, *n*-hexyl 2-cyanoacrylate, *n*-heptyl 2-cyanoacrylate, *n*-octyl 2-cyanoacrylate, *2*-octyl 2-cyanoacrylate, 2-ethylhexyl 2-cyanoacrylate, tetrahydrofurfuryl 2-cyanoacrylate, and mixtures thereof, preferably it is selected from the group consisting of ethyl 2-cyanoacrylate, 2-methoxyethyl 2-cyanoacrylate, *n*-butyl 2-cyanoacrylate, *n*-hexyl 2-cyanoacrylate, 2-ethylhexyl 2-cyanoacrylate, 2-octyl 2-cyanoacrylate and mixtures thereof.

3. The cyanoacrylate adhesive composition according to claim 1 or 2, **characterized in that** the multicyanoacrylate is a bis-cyanoacrylate monomer.

4. The cyanoacrylate adhesive composition according to claim 3, **characterized in that** the bis-cyanoacrylate monomer is selected from methylene glycol bis(2-cyanoacrylate), ethylene glycol bis(2-cyanoacrylate), 1,3-propanediol bis(2-cyanoacrylate), 1,3-butanediol bis(2-cyanoacrylate), 1,4-butanediol bis(2-cyanoacrylate), *trans*-2-Butene-1,4-diol bis(2-cyanoacrylate), 1,4-cyclohexanedimethanol bis(2-cyanoacrylate), 1,5-pentanediol bis(2-cyanoacrylate), 1,6-hexanediol bis(2-cyanoacrylate), 2,5-hexanediol bis(2-cyanoacrylate), 1,7-heptanediol bis(2-cyanoacrylate), 1,8-octanediol bis(2-cyanoacrylate), 1,9-nonanediol bis(2-cyanoacrylate), 1,10-decanediol bis(2-cyanoacrylate),1,12-dodecanediol bis(2-cyanoacrylate), 2-butyne-1,4-diol bis(2-cyanoacrylate), neopentyl glycol bis(2-cyanoacrylate), 1,4-bis(hydroxymethyl) benzene bis(2-cyanoacrylate), 1,3-bis(hydroxymethyl) tetramethyl-disiloxane bis(2-cyanoacrylate), or bis(4-hydroxybutyl) ether bis(2-cyanoacrylate), preferably it is 1,6-hexanediol bis(2-cyanoacrylate).

5. The cyanoacrylate adhesive composition according to any of claims 1 to 4, **characterized in that** the unsaturated carboxylic acid anhydride is selected from maleic anhydride, itaconic anhydride, phthalic anhydride, isophthalic anhydride, and mixtures thereof.

6. The cyanoacrylate adhesive composition according to claim 1, **characterized in that** the content of multicyanoacrylate monomer, preferably 1,6-hexanediol bis(2-cyanoacrylate), is 0.6 wt% and the unsaturated carboxylic acid anhydride is a combination of maleic anhydride and phthalic anhydride, wherein the content of maleic anhydride is comprised between 0.05 wt% and 0.5 wt% and the content of phthalic anhydride is between 1.0 wt% and 2.0 wt%, wherein the wt% is based on the total weight of the monomer composition.

7. The cyanoacrylate adhesive composition according to any of claims 1 to 6, **characterized in that** the cyanoacrylate composition is either one-component or two-component.

8. The cyanoacrylate adhesive composition according to any of claims 1 to 7, **characterized in that** the cyanoacrylate composition is either non-photocurable or photocurable.

9. The cyanoacrylate adhesive composition according to claim 1, **characterized in that** the cyanoacrylate composition is non-photocurable one-component, wherein the content of cyanoacrylate monomer is comprised between 96.4 wt% and 99 wt%, the content of multicyanoacrylate monomer, preferably 1,6-hexanediol bis(2-cyanoacrylate), is comprised between 0.5 wt% and 0.8 wt%, the content of maleic anhydride is comprised between 0.05 wt% and 0.5 wt% and the content of phthalic anhydride is between 1.0 wt% and 2.0 wt%, wherein the wt% is based on the total weight of the monomer composition.

10. The cyanoacrylate adhesive composition according to any of claims 1 to 9, **characterized in that** it further comprises radical polymerization inhibitors and acid stabilizing agents.

11. The cyanoacrylate adhesive composition according to any of claims 1 to 10, **characterized in that** it further comprises additives selected from thickening agents, plasticizers, thixotropic agents, crosslinking agents, accelerating agents, photoinitiators, ferrocene, initiator, and mixtures thereof.

12. A syringe or a cartridge comprising the cyanoacrylate adhesive composition of any of claims 1 to 11.

13. The use of the cyanoacrylate adhesive composition of any of claims 1 to 11 for bonding substrates.

14. A method for bonding substrates, **characterized in that** it comprises the steps of:
1) applying the cyanoacrylate adhesive composition of any of claims 1 to 11 to at least one of the substrates,
2) placing together the substrates,
3) optionally irradiating with a light of a wavelength comprised between 380 nm and 415 nm, and
4) maintaining the substrates clamped up to fixation.

15. The cyanoacrylate adhesive composition according to any of claims 1 to 11 for use as tissue adhesive (bioadhesive) and biosealant.
